# FASCICULE DE BREVET EUROPEEN

(11) **EP 0 854 735 B1**
(45) Date de publication et mention de la délivrance du brevet: **22.03.2000**
(21) Numéro de dépôt: 96933507.4
(22) Date de dépôt: 09.10.1996
(51) Int. Cl.: A61M 5/30

(54) **DISPOSITIF D'INJECTION PAR JET SANS AIGUILLE, COMPORTANT UNE CARTOUCHE SURMOULEE**
NADELLOSE STRAHLINJEKTIONSVORRICHTUNG MIT ÜBERGEGOSSENER AMPULLE
NEEDLELESS JET INJECTION DEVICE COMPRISING A MOULDED-ON CARTRIDGE

(30) Priorité: 09.10.1995 FR 9511872
(43) Date de publication de la demande: 29.07.1998
(73) Titulaire: Moreau Defarges, Alain, 75016 Paris (FR); Moreau Defarges, Xavier, 92150 Suresnes (FR)
(72) Inventeur: Moreau Defarges, Alain, 75016 Paris (FR); Moreau Defarges, Xavier, 92150 Suresnes (FR)
(74) Mandataire: Armengaud Ainé, Alain
(86) Numéro de dépôt international: FR9601573
(87) Numéro de publication internationale: WO9713536

(56) Documents cités:
- EP-A- 0 427 457
- WO-A-95/03844
- WO-A-95/27523
- WO-A-96/15821
- GB-A- 677 523
- US-A- 3 688 765

## Description

La présente invention est relative à un dispositif permettant l'injection de produit, notamment pharmaceutique, dans le corps humain ou animal.

Elle vise plus particulièrement un appareil dépourvu d'aiguille hypodermique, équipé d'un système d'armement, qui autorise l'administration par voie sous-cutanée, intradermique ou intramusculaire, de substances médicamenteuses ou vaccinales, contenues dans une dose, à usage unique, formant cartouche.

On connaît des appareils d'injection par jet transcutané sans aiguille, qui ont été développés pour la médecine vétérinaire ou humaine. L'absence d'aiguille simplifie l'utilisation de l'appareil et ne requiert pas de connaissances spécifiques de l'usager. Ils sont généralement utilisés pour des campagnes massives de vaccination et sont conçus de manière à être mis en oeuvre rapidement par un personnel non spécialiste. Le recours à un jet évite au maximum la contamination bactérienne et/ou virale d'un sujet à l'autre, qui se produisait dans les cas où une même aiguille est utilisée sans stérilisation par plusieurs sujets. Ces appareils sont généralement conformés sous forme de pistolet, équipé d'un récipient de produit à injecter, véhiculé au travers d'une buse, par l'action d'un piston se déplaçant dans une chambre, préalablement remplie de la substance, le piston étant mû par un percuteur. En variante, le pistolet comporte une culasse ou magasin renfermant une cartouche placée également dans l'axe du percuteur.

Ainsi, on connaît par le document WO 95/03844 un dispositif d'injection par jet sans aiguille qui est pourvu d'un dispositif d'armement actionné par la rotation d'un capuchon situé à l'une des extrémités du corps dudit dispositif.

Le document WO 95/27523 décrit un dispositif d'injection par jet sans aiguille pourvu à l'une de ses extrémités d'une cartouche réalisée d'une seule pièce.

Le document WO 96/15821 décrit une cartouche comportant une capsule sur laquelle est monté en force un manchon qui induit des contraintes sur la capsule.

Enfin, le document EP 427 457 décrit un dispositif d'injection par jet sans aiguille pourvu d'une cartouche qui coopère à l'une des extrémités du dispositif, par l'intermédiaire d'une pièce formant culasse.

Compte tenu du mode d'action du percuteur qui possède une énergie cinétique importante avant d'atteindre le piston de la cartouche qui peut, dans le cas où la cartouche n'est pas correctement disposée dans le magasin du pistolet, provoquer son éclatement au début de l'injection, ces appareils ne sont pas fiables et ne sont pas d'une mise en oeuvre aisée pour une utilisation ne relevant pas d'un usage fréquent.

La présente invention vise donc à remédier à ces inconvénients, en proposant un dispositif dépourvu de magasin pour cartouche et permettant l'injection par jet sans aiguille de produit contenu dans une cartouche directement placée en tête dudit dispositif, dans des conditions d'asepsie rigoureuses, pour un usage individuel.

Il est à noter que seuls les modes de réalisation pourvus d'une cartouche d'injection réalisées par surmoulage sont couverts par les revendications.

A cet effet, la cartouche d'injection d'un produit notamment pharmaceutique par jet sans aiguille à usage unique, destinée à être fixée à une première extrémité du corps d'un dispositif d'injection, la seconde extrémité du corps recevant un capuchon pouvant, grâce à un mouvement relatif du corps, entraîner un dispositif d'armement, coopérant avec un organe de percussion destiné à coopérer avec la cartouche, cette cartouche comprenant deux éléments dont le premier élément en verre réalise le récipient devant contenir ledit produit, caractérisée en ce que le second est constitué par une enveloppe en matière plastique qui surmoule ledit premier élément et en ce que ladite cartouche comporte à l'une de ses extrémités, des moyens permettant l'engagement et la solidarisation de ladite cartouche sur le nez du dispositif d'injection.

D'autres caractéristiques et avantages de la présente invention ressortiront de la description faite ci-après, en référence aux dessins annexés qui en illustrent un exemple de réalisation dépourvu de tout caractère limitatif. Sur les figures :

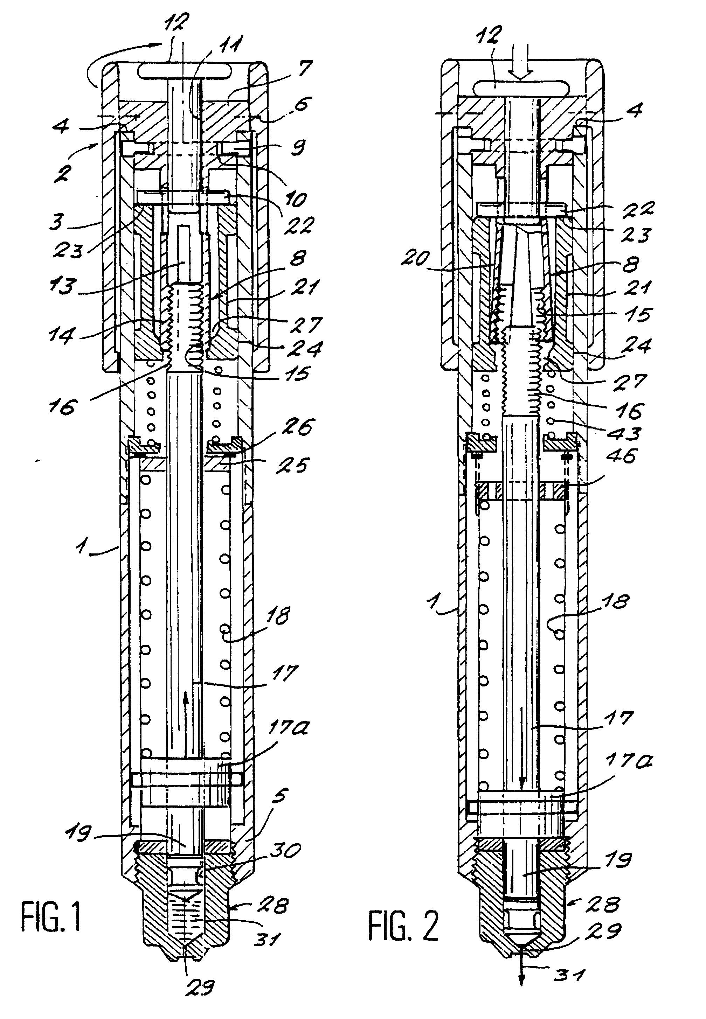

- la figure 1 est une vue en plan, en élévation frontale et en coupe, du dispositif selon l'invention, en position armée ;
- la figure 2 est une vue en plan, en élévation frontale et en coupe, du dispositif selon l'invention, en position déclenchée;

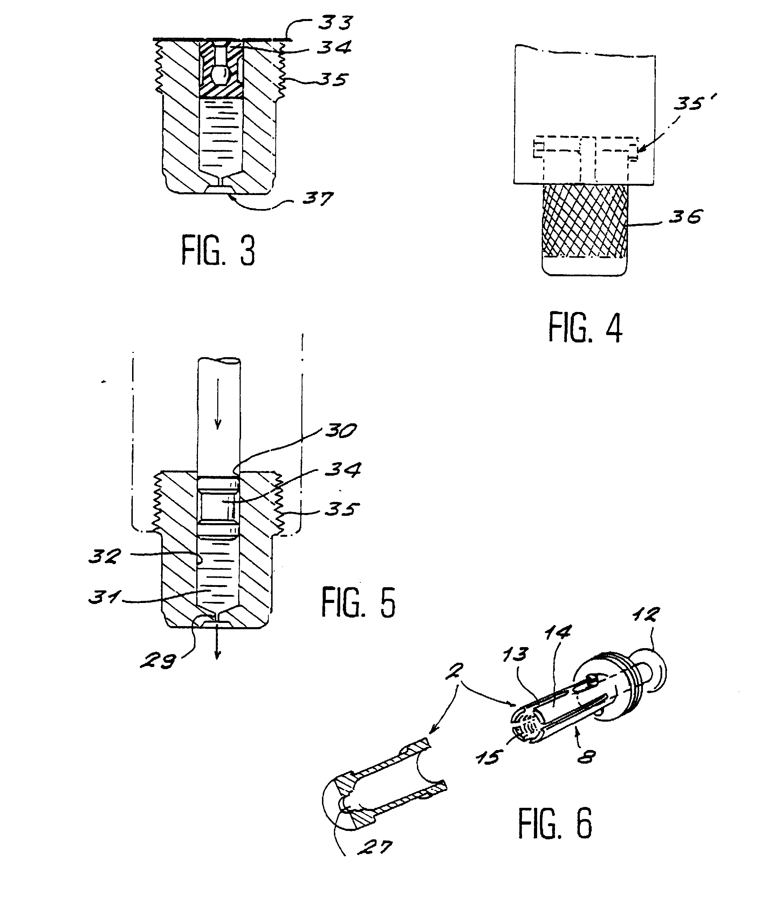

- la figure 3 est une vue à plus grande échelle et en coupe, d'une cartouche contenant une dose de produit injectable ;
- la figure 4 est une vue illustrant un autre mode de fixation de la cartouche sur le dispositif ;
- la figure 5 est une vue en coupe illustrant le mouvement du percuteur sur le bouchon en élastomère contenu dans la cartouche ;
- la figure 6 est une vue en perspective du système de pince devant emprisonner la tige du percuteur ;

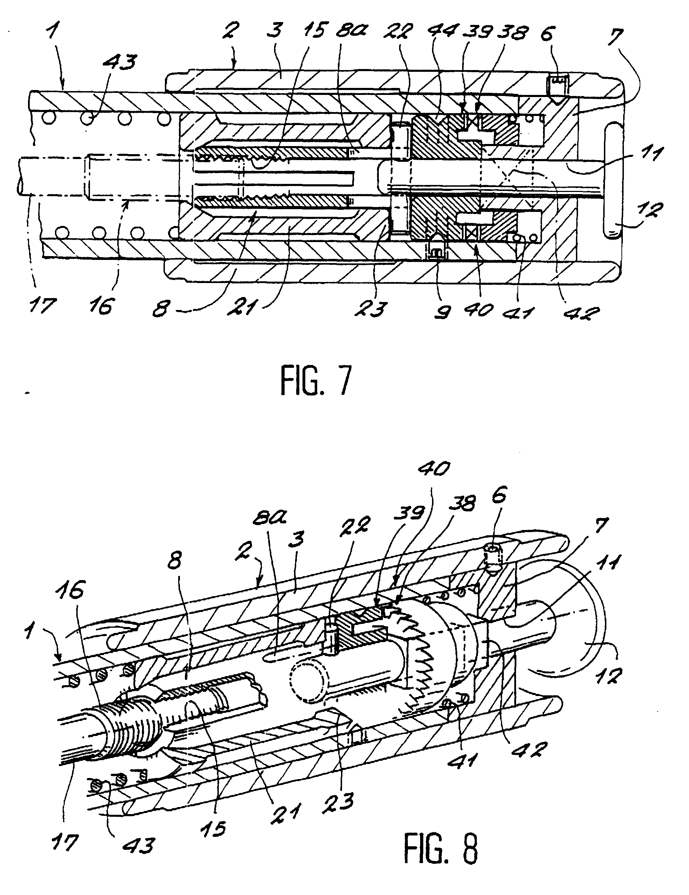

- la figure 7 est une vue en plan, en élévation frontale et en coupe, du dispositif selon un autre mode de l'invention, comportant un organe de liaison débrayable ;
- la figure 8 est une vue en perspective de l'organe de liaison débrayable ;

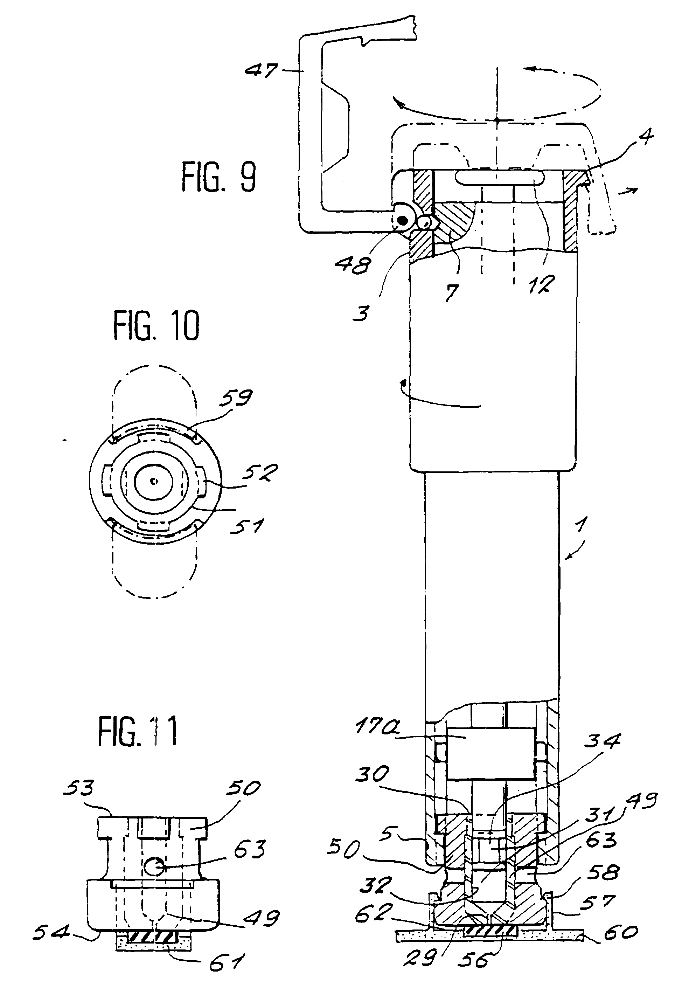

- la figure 9 est une vue en plan, en élévation frontale et en coupe, du dispositif selon l'invention ;
- la figure 10 est une vue en plan, en élévation frontale et en coupe, d'une cartouche selon un second mode de réalisation ;
- la figure 11 est une vue en plan, en élévation latérale et en coupe, d'une cartouche selon un second mode de réalisation.

Selon un mode préféré de réalisation, le dispositif objet de l'invention comprend essentiellement un corps 1 s'étendant sous la forme d'un tube, notamment de section droite cylindrique, destiné à recevoir un dispositif d'armement 2 lié à un capuchon 3, notamment moleté afin d'améliorer sa préhension par l'utilisateur, prévu à l'une des extrémités 4, 5 dudit corps. Le capuchon est réalisé à partir d'une portion de tube, de section similaire à celle du corps principal dudit dispositif, dont la diamètre est cependant supérieur afin d'en permettre un mouvement relatif de rotation autour du corps et coopère par l'intermédiaire d'un dispositif d'accrochage 6, notamment du type clavette, à une bague 7, elle-même solidaire d'une pince 8. Ladite bague est emmanchée avec jeu à l'extrémité du corps 1 et peut tourner librement dans ce dernier, sa position axiale dans le tube étant déterminée grâce à une butée radiale 9 à la paroi du tube et débouchant dans une rainure circulaire 10 pratiquée sur ladite bague 7 et positionnée en regard de la butée 9. Le mouvement de rotation issu du capuchon 3 est transmis par l'intermédiaire du dispositif d'accrochage 6 à la bague.

Ladite bague 7 est pourvue en outre d'un évidement central 11 pour le passage d'un percuteur 12, libre en translation axiale et débouchant à l'intérieur de ladite pince 8. Cette pince constitue l'un des maillons du dispositif d'armement. De forme globalement cylindrique, elle est munie d'une pluralité de découpes 13 orientées sensiblement parallèlement à l'axe du corps de manière à conformer une pluralité de pattes flexibles 14. On prévoit en outre de tarauder l'alésage 15 intérieur de ladite pince 8, de manière à conformer des empreintes aptes à se visser autour d'une portion filetée 16 prévue sur une tige 17 formant percuteur. Le pas du filetage est déterminé en fonction de la démultiplication choisie et de la constante de raideur d'un ressort 18. L'intensité du percuteur peut également être ajustée à l'aide d'un pré-réglage de l'écrasement du ressort, l'espace inter-spire pouvant être réglé par l'intermédiaire d'une molette 46 se déplaçant sur la tige 17 et en contact de 18 ou par l'intermédiaire d'un empilement de rondelles disposées entre le ressort 18 et d'une bague 25. Les spires du ressort 18 sont comprimées par le mouvement relatif de rotation entre le capuchon 3 et le corps 1.

Selon une caractéristique avantageuse, le capuchon 3 comporte un dispositif de sécurité 47 qui recouvre la tige du percuteur 12. Ce dispositif de sécurité 47 est relié au capuchon 3 par l'intermédiaire d'une articulation 48 qui autorise un mouvement de rotation de ce dernier 47, entre une position verrouillée et une position déverrouillée de la tige du percuteur 12. La rotation du dispositif de sécurité 47 permet également d'agir sur l'organe d'accrochage 6 entre le capuchon 3 et la bague 7. En effet, il est impératif que l'utilisateur ne puisse armer le dispositif de percussion que lorsque le dispositif de sécurité 47 recouvre la tige du percuteur 12 ; c'est pourquoi, lorsque le dispositif de sécurité 47 est verrouillé sur le capuchon 3, il agit sur l'organe d'accrochage 6 de manière à ce que celui-ci autorise la transmission du mouvement de rotation entre le capuchon 3 et la bague 7.

Lorsque le dispositif de sécurité 47 est déverrouillé, c'est-à-dire lorsqu'il ne recouvre plus la tige du percuteur 12, il n'agit plus sur l'organe d'accrochage 6 et l'utilisateur ne peut armer le dispositif de percussion.

On dispose donc une tige 17, formant piston, à l'intérieur dudit corps 1, sa longueur étant choisie de manière telle que son extrémité 19, non liée à ladite pince 8, affleure l'une des surfaces frontales 4, 5 du corps 1, lorsque le dispositif d'armement 2 est opérationnel.

L'enveloppe 20 périphérique de la pince est enserrée par une entretoise 21, également de section similaire à celle du corps, dont le diamètre extérieur correspond au diamètre intérieur du corps, de manière à conformer des zones de guidage. Sa position axiale à l'intérieur du corps étant limitée d'une part, à l'une de ses extrémités 23, 24, par une goupille 22 traversant de part en part et radialement le percuteur 12, et d'autre part son autre extrémité frontale constituant une surface d'appui pour un ressort 43 ; la position axiale du ressort et son autre surface d'appui étant en outre limitées par exemple par une bague 25 insérée dans le corps, solidaire de ladite tige 17 et immobilisée axialement éventuellement par un circlips 26, ou par un épaulement pratiqué sur la tige.

En position armement, l'utilisateur imprime un mouvement relatif de rotation entre le corps 1 et le capuchon 3 ; comme nous l'avons vu précédemment, le mouvement de rotation du capuchon se transmet à la fois à ladite bague 7 et à la pince 8 solidaire de cette dernière ; les quelques filets de ladite pince 8 en prise avec ceux de la tige permettent d'initier le mouvement de translation entre la tige 17 (formant vis) et la pince 8 (formant écrou) et le rapprochement relatif de ces deux pièces. Ce mouvement de translation comprime les spires du ressort 18 jusqu'à ce que l'espace inter-spires soit réduit au maximum. Selon un autre mode de réalisation, le ressort à spires peut être remplacé par un ressort hélicoïdal ou par tout autre dispositif élastique.

En position désarmement ou déclenchement, l'utilisateur imprime une légère poussée sur le percuteur 12, la goupille 22 faisant saillie radialement et au contact de l'une des faces frontales 23, 24 de l'entretoise, transmet un mouvement relatif de translation entre l'entretoise 21 et la pince 8, ce qui dégage l'extrémité de celle-ci d'une portée conique 27 prévue au fond de l'alésage de ladite entretoise 21. La poussée du ressort 18 combinée à la flexibilité des pattes de la pince libère les filets respectifs préalablement en prise, provoquant ainsi un mouvement de translation, quasiment impulsionnel de l'ensemble de la tige.

Selon un autre mode de réalisation de l'invention, on interpose, entre le capuchon 3 et l'organe d'armement solidaire de la pince 8 contenue dans le corps 1, un organe de liaison 38 ne permettant le mouvement relatif de rotation entre ces deux pièces que dans un seul sens de rotation. En effet, pour éviter toute détérioration de l'appareil et pour en simplifier la manipulation par l'utilisateur, on dispose d'un organe de liaison 38 débrayable se composant principalement de deux pièces 39, 40 notamment de section circulaire. L'une des pièces 39 est solidaire du capuchon 3, tandis que l'autre 40 est liée à la pince 8 et au corps 1 par l'intermédiaire d'une gorge 44, chacune des pièces dispose par ailleurs au niveau de leur face en contact d'une pluralité de zones 41 en relief. Ces zones 41 possèdent un profil apte à permettre d'une part un glissement relatif entre les pièces 39, et 40 selon un sens de rotation entre le capuchon 3 et le corps 1 et d'autre part à transmettre le couple dans l'autre sens de rotation du capuchon 3 par rapport au corps 1.
Préférentiellement, les zones 41 en relief sont réalisées à partir d'une pluralité de dents notamment à section droite triangulaire.

Pour que l'organe de liaison 38 fonctionne, il convient cependant de ne pas supprimer totalement les mouvements des pièces 39 et 40 par rapport au capuchon 3 et à la pince 8 ; ainsi lorsque l'organe de liaison est actif (débrayé) il faut que les pièces 39, 40 s'échappent longitudinalement l'une par rapport à l'autre et que ces pièces reviennent dans leur position d'engrènement après suppression du couple ; on interpose donc au droit de la surface extérieure de l'une des pièces 39 ou 40 un organe élastique 41, notamment du type ressort, au niveau d'une portion de guidage 42, qui compense les mouvements de translation entre les pièces.

Selon une autre caractéristique de l'invention, on dispose à l'autre extrémité 5 du corps 1 dudit dispositif, une cartouche 28 par des moyens connus tels que notamment par vissage, par enclipsage (douille, baïonnette).

Selon une caractéristique avantageuse de l'invention, la cartouche 28 comporte deux éléments 49, 50 :
- le premier élément 49 réalise le récipient devant contenir le principe actif et il est particulièrement obtenu à partir d'un verre de qualité médicale de type I, car le récipient doit contenir des produits injectables ;
- le second élément 50 est constitué par une enveloppe en matière plastique qui recouvre sur ledit premier élément 49.

Avantageusement, la matière plastique utilisée dans l'opération de surmoulage, c'est-à-dire de recouvrement, doit pouvoir résister à une opération de stérilisation à haute température (aux environs de 120 °C), approuvée pour des applications pharmaceutiques, ne pas être sensible aux écarts de température au niveau de sa dilatation, étant donné que les cartouches 28 sont généralement stockées dans une ambiance dont la température est comprise entre 2 et 10 °C. Par ailleurs, après surmoulage sur le premier élément 49, la matière plastique employée est translucide, voire transparente et peut être teintée. On choisira par exemple comme matière plastique, le produit appelé "TPX" (polyméthylpentène).

Le premier élément 49 en verre, globalement de forme sensiblement cylindrique, comporte à chaque extrémité un orifice 29, 30. L'un 30 des orifices possède un diamètre sensiblement équivalent au diamètre de la tige 17, tandis que l'autre 29, de petit diamètre, notamment de l'ordre de quelques dixièmes de millimètre, sert de buse. La cavité interne de ladite cartouche est remplie sous vide d'un principe actif 31 et est éventuellement revêtue par un film 32 de matière, compatible avec les propriétés physico-chimiques dudit produit afin de limiter au maximum le phénomène d'adsorption.

La cartouche 28 ainsi formée par le surmoulage d'une matière plastique sur un premier élément 49 en verre, comporte à l'une de ses extrémités, des moyens 51 permettant l'engagement et la solidarisation de ladite cartouche sur le nez du dispositif d'injection.

Avantageusement, les moyens 51 permettant l'engagement et la solidarisation sont réalisés lors de l'opération de surmoulage par l'intermédiaire d'une pluralité d'ergots 52 faisant saillie radialement et disposés selon le diamètre de ladite cartouche 28, ces ergots coopérant au niveau des baïonnettes prévues à l'extrémité 53 du dispositif d'injection.

L'autre extrémité 54 de ladite cartouche 28 est conformée en une surface plane destinée à être appliquée contre la surface de la peau de l'utilisateur.

L'orifice de sortie 31 ménagé sur cette extrémité 54 doit être protégé d'éventuelles contaminations du milieu environnant et à cette fin, on dispose un capuchon 55 pourvu en son centre d'un joint en élastomère 56, notamment en silicone.

Selon un autre mode de réalisation, on dispose sur l'extrémité 54 une pellicule recouvrant la surface de la cartouche 28.

Ce capuchon 55 est obtenu par un procédé de moulage de matière plastique, éventuellement similaire à celle composant la cartouche.

Le capuchon 55 dispose avantageusement de parois latérales 57 qui viennent enserrer l'extrémité de ladite cartouche 28, lesdites parois 57 étant pourvues à leurs extrémités de zones en relief 58 autorisant leur clipsage au niveau d'empreintes 59 prévues sur les parois latérales extérieures de ladite cartouche 28, assurant de ce fait le maintien du capuchon 55 sur la cartouche.

La paroi supérieure 60 du capuchon 55 forme une zone de préhension qui permet à l'utilisateur de positionner les ergots 52 de la cartouche 28 au droit des baïonnettes du dispositif d'injection et de par un simple mouvement de rotation, de bloquer les ergots 52 dans les baïonnettes et de permettre, lorsque l'utilisateur continue d'imprimer au capuchon 55 et donc à la cartouche 28 qui lui est solidaire ce mouvement de rotation après blocage des ergots 52, de désolidariser les parois latérales 57 du capuchon 55 des empreintes 59 de la cartouche 28, de manière à mettre le dispositif d'injection prêt à l'emploi.

Selon une première variante, le joint en élastomère 56 est obtenu lors d'une opération simultanée de moulage du capuchon 55.

Selon une seconde variante, le joint en élastomère 56 est rapporté et monté en force dans un orifice 61 prévu sur la paroi interne 62 du capuchon 55.
Selon une autre caractéristique avantageuse de l'invention, on prévoit de disposer dans l'épaisseur du surmoulage en plastique de la cartouche 28 une pluralité de manque de matière plastique afin de réaliser des lumières 63 pour améliorer la visibilité au niveau du premier élément 49 en verre. La cartouche 28 ainsi obtenue est d'un usage unique et donc jetable.

Après remplissage, on procède à la fermeture de l'orifice 30 de la cartouche par un opercule 33 afin de garantir des conditions satisfaisantes d'asepsie. On prévoit par ailleurs d'interposer entre la dose de produit contenue dans la cavité de la cartouche et l'opercule d'étanchéité, un bouchon en élastomère 34 pour la fermeture de la dose, celui-ci devant communiquer au liquide la pression exercée par le piston d'un percuteur.

Selon une caractéristique avantageuse, on prévoit que le piston du percuteur soit enfoncé de quelques millimètres dans le corps de la cartouche afin que l'effort de percussion soit dirigé et centré dans l'axe du bouchon en élastomère 34 pour éviter tout risque d'éclatement du premier élément en verre formant la cartouche 28.

Selon un autre mode d'utilisation de la cartouche 28, celle-ci est vide et est remplie par l'utilisateur juste avant usage.

L'enveloppe extérieure de ladite cartouche dispose par ailleurs, d'une part de moyens de fixation 35, 35' audit corps (pas de vis, picot...), et d'autre part de zones en relief 36 pour sa préhension par l'utilisateur. La face frontale correspondant à la buse comprend éventuellement une cuvette 37 dont la profondeur est variable mais garantit que le jet sortant de l'orifice de la buse possède le temps nécessaire à son établissement hydrodynamique avant l'injection sous-cutanée, intradermique ou intramusculaire.

L'invention telle qu'elle est décrite ci-dessus est d'une grande facilité d'emploi : il n'y a plus de stérilisation, ni de lavage de l'appareil, tout en garantissant une sûreté accrue pour l'utilisateur de par l'absence d'aiguille et l'impossibilité d'exécuter une réutilisation sans avoir préalablement rechargé par une nouvelle dose dans l'appareil, le mode de fixation de la cartouche évitant tous les risques d'éclatement de cette dernière de par l'absence de culasse. Cette invention est avantageusement adaptée à un usage par un utilisateur individuel ne possédant pas de connaissances spécifiques en matière d'injections sous-cutanées, intradermiques ou intramusculaires, elle réduit d'une part les risques d'accidents, toujours possibles avec un moyen d'injection à aiguille et d'autre part, elle supprime la peur de la piqûre ainsi que tout risque de contamination. L'utilisation de ce dispositif permet de plus de respecter la chronobiologie du patient. Cette invention trouve des développements avantageux dans l'injection d'une dose de faible volume, pouvant atteindre notamment de 0,05 à 0,2 ml. Elle présente un intérêt tout particulier pour l'administration de médicaments ou de vaccins chez l'homme ou l'animal. On citera, entre autres produits, des polypeptides ou des peptides, tels que des enzymes et tout spécialement la calcitonine, utilisée pour la prévention de la perte osseuse et le traitement de l'ostéoporose ou encore des médicaments contre la migraine. D'autres produits, en particulier des polypeptides ou des peptides, administrables par le dispositif objet de l'invention, comprennent des hormones, comme l'insuline, la somatostatine, l'hormone de croissance, des facteurs de la coagulation, par exemple des facteurs anti-hémophiliques, des composants du plasma, comme l'erythropoïétine, des polypeptides anti-viraux, comme les interférons ou des immuno-modulateurs, comme les lymphokines. Ce dispositif est également spécialement approprié pour l'administration de préparations vaccinales.

Il demeure bien entendu que la présente invention n'est pas limitée aux exemples de réalisation décrits et représentés ci-dessus, mais qu'elle en englobe toutes les variantes. Ainsi, les cartouches peuvent être présentées devant la tige du percuteur à l'aide d'un barillet ou d'un chargeur, elles peuvent également comprendre la dose du produit à injecter.

## Revendications

1. Cartouche d'injection (28) d'un produit notamment pharmaceutique par jet sans aiguille à usage unique, destinée à être fixée à une première extrémité (5) du corps (1) d'un dispositif d'injection, la seconde extrémité (4) du corps (1) recevant un capuchon (3) pouvant, grâce à un mouvement relatif du corps (1), entraîner un dispositif d'armement (2), coopérant avec un organe de percussion (17) destiné à coopérer avec la cartouche (28), cette cartouche comprenant deux éléments (49, 50) dont le premier élément (49) en verre réalise le récipient devant contenir ledit produit et comporte à l'une de ses extrémités, des moyens (51) permettant l'engagement et la solidarisation de ladite cartouche sur le nez du dispositif d'injection, caractérisée en ce que le second (50) est constitué par une enveloppe en matière plastique qui surmoule ledit premier élément (49).

2. Cartouche d'injection selon la revendication 1, caractérisée en ce que le premier élément (49) est obtenu à partir d'un verre de qualité médicale de type I.

3. Cartouche d'injection selon la revendication 1, caractérisée en ce que le second élément (50) est réalisé en une matière plastique possédant les caractéristiques suivantes :
- résister à une opération de stérilisation à haute température,
- être approuvée pour des applications pharmaceutiques,
- être insensible aux écarts de température,
- être translucide, voire transparente et éventuellement teintée.

4. Cartouche d'injection selon la revendication 1, caractérisée en ce que le premier élément (49), globalement de forme sensiblement cylindrique, comporte à chaque extrémité un orifice (29, 30), l'un (30) des orifices possédant un diamètre sensiblement équivalent au diamètre d'une tige (17) de l'organe de percussion, tandis que l'autre (29), de petit diamètre, notamment de l'ordre de quelques dixièmes de millimètre, sert de buse.

5. Cartouche d'injection selon la revendication 1, caractérisée en ce que les moyens (51) permettant l'engagement et la solidarisation sont réalisés lors de l'opération de recouvrement du surmoulage par l'intermédiaire d'une pluralité d'ergots (52) faisant saillie radialement et disposés selon le diamètre de ladite cartouche (28), ces ergots coopérant au niveau des baïonnettes prévues à l'extrémité (53) du dispositif d'injection.

6. Cartouche d'injection selon l'une quelconque des revendications précédentes, caractérisée en ce que l'extrémité (54) de la cartouche (28) est revêtue d'un capuchon (55) pourvu en son centre d'un joint en élastomère (56), notamment en silicone.

7. Cartouche d'injection selon la revendication 6, caractérisée en ce que le capuchon (55) dispose de parois latérales (57) qui viennent enserrer l'extrémité de ladite cartouche (28), lesdites parois (57) étant pourvues à leurs extrémités de zones en relief (58) autorisant leur clipsage au niveau d'empreintes (59) prévues sur les parois latérales extérieures de ladite cartouche (28), assurant de ce fait le maintien du capuchon (55) sur la cartouche.

8. Cartouche d'injection selon l'une des revendications 6 ou 7, caractérisée en ce que la paroi supérieure (60) du capuchon (55) forme une zone de préhension qui permet à l'utilisateur de positionner les ergots (52) de la cartouche (28) au droit des baïonnettes du dispositif d'injection.

9. Cartouche d'injection selon la revendication 6, caractérisée en ce que le joint en élastomère (56) est obtenu lors d'une opération simultanée de moulage du capuchon (55).

10. Cartouche d'injection selon la revendication 6, caractérisée en ce que le joint en élastomère (56) est rapporté et monté en force dans un orifice (61) prévu sur la paroi interne (62) du capuchon (55).

11. Cartouche d'injection selon l'une quelconque des revendications précédentes, caractérisée en ce qu'elle comporte une pluralité de manque de matière plastique, pratiqué dans l'épaisseur du surmoulage en plastique de la cartouche (28), afin de réaliser des lumières (63) pour améliorer la visibilité au niveau du premier élément (49).

## Patentansprüche

1. Injektionsampulle (28) zum einmaligen Gebrauch zum Spritzen eines vor allem pharmazeutischen Produkts ohne Nadel, die an einem ersten Ende (5) des Körpers (1) einer Injektionsvorrichtung befestigt wird, wobei das zweite Ende (4) des Körpers (1) eine Kappe (3) aufnimmt, die durch eine relative Bewegung des Körpers (1) eine Armierungsvorrichtung (2) mitnimmt, die mit einem Schlagelement (17) zusammenwirkt, das mit der Ampulle (28) zusammenwirkt, wobei diese Ampulle zwei Elemente (49, 50) aufweist, von denen das erste (49) aus Glas den Behälter bildet, der das Produkt enthält, und an einem ihrer Enden eine Vorrichtung (51) aufweist, die das Einführen und die Befestigung der Ampulle an der Nase der Injektionsvorrichtung ermöglicht, dadurch gekennzeichnet, daß das zweite Element (50) durch einen Kunststoffmantel gebildet ist, der über das erste Element (49) geformt ist.

2. Injektionsampulle nach Anspruch 1, dadurch gekennzeichnet, daß das erste Element (49) aus einem Glas medizinischer Qualität vom Typ I ausgebildet ist.

3. Injektionsampulle nach Anspruch 1, dadurch gekennzeichnet, daß das zweite Element (50) aus einem Kunststoffinaterial ausgebildet ist, das folgende Eigenschaften aufweist:
- hitzebeständig für einen Sterilisationsvorgang bei hoher Temperatur
- zugelassen für pharmazeutische Anwendungen
- unempfindlich gegenüber Temperaturschwankungen
- durchscheinend, ja sogar durchsichtig und möglicherweise getönt.

4. Injektionsampulle nach Anspruch 1, dadurch gekennzeichnet, daß das erste Element (49), das im wesentlichen zylindrische Form aufweist, an jedem Ende eine Öffnung (29, 30) aufweist, wobei eine (30) dieser Öffnungen einen Durchmesser aufweist, der im wesentlichen äquivalent zum Durchmesser eines Stiftes (17) des Schlagelements ist, während die andere Öffnung (29), die einen kleineren Durchmesser hat, besonders in der Größenordnung von einigen Zehntelmillimetern, als Düse dient.

5. Injektionsampulle nach Anspruch 1, dadurch gekennzeichnet, daß die Vorrichtung (51), die das Einführen und die Befestigung gewährleistet, während der Abdeckung durch Überformen durch eine Vielzahl von Vorsprüngen (52) gebildet wird, die radial hervorstehen und auf dem Durchmesser der Ampulle (28) angeordnet sind, wobei die Vorsprünge mit der am Ende (53) der Injektionsvorrichtung vorgesehenen Bajonettvorrichtung zusammenwirken.

6. Injektionsvorrichtung nach einem der vorangehenden Ansprüche, dadurch gekennzeichnet, daß das Ende (54) der Ampulle (28) eine Kappe (55) aufweist, die in ihrer Mitte eine Dichtung (56) aus einem Elastomer, besonders aus Silikon aufweist.

7. Injektionsvorrichtung nach Anspruch 6, dadurch gekennzeichnet, daß die Kappe (55) Seitenwände (57) aufweist, die das Ende der Ampulle (28) einklemmen, wobei die Wände (57) an ihren Enden Reliefbereiche (58) aufweisen, die ein Anclipsen an an den äußeren Seitenwänden der Ampulle (28) vorgesehenen Vertiefungen (59) ermöglichen, wodurch die Kappe (55) auf der Ampulle gehalten wird.

8. Injektionsampulle nach einem der Ansprüche 6 oder 7, dadurch gekennzeichnet, daß die obere Wand (60) der Kappe (55) einen Griffbereich bildet, der es dem Benutzer ermöglicht, die Vorsprünge (52) der Ampulle (28) direkt über der Bajonettvorrichtung der Injektionsvorrichtung zu positionieren.

9. Injektionsampulle nach Anspruch 6, dadurch gekennzeichnet, daß die Elastomerdichtung (56) gleichzeitig mit dem Formen der Kappe (55) hergestellt wird.

10. Injektionsampulle nach Anspruch 6, dadurch gekennzeichnet, daß die Elastomerdichtung (56) mit Kraft in eine an der Innenwand (62) der Kappe (55) vorgesehene Öffnung (61) eingesetzt und montiert wird.

11. Injektionsampulle nach einem der vorangehenden Ansprüche, dadurch gekennzeichnet, daß sie eine Vielzahl an Löchern im Kunststoffmaterial aufweist, die in der Dicke der Kunststoffüberformung der Ampulle (28) ausgebildet sind und Sichtöffnungen (63) bilden, um die Sichtbarkeit auf der Höhe des ersten Elements (49) zu verbessern.

## Claims

1. Single-use needleless cartridge (28) for the injection of a product, in particular a pharmaceutical product, by a jet, the cartridge being intended to be fixed to a first end (5) of the body (1) of an injection device, the second end (4) of the body (1) receiving a cap (3) which is able, by virtue of a relative movement of the body (1), to drive a cocking device (2), cooperating with a percussion member (17) intended to cooperate with the cartridge (28), this cartridge comprising two members (49, 50) whereof the first member (49) made of glass forms the container intended to contain said product and comprises at one of its ends, means (51) allowing the engagement and interconnection of said cartridge on the nose of the injection device, characterised in that the second member (50) is constituted by a casing of plastics material which is moulded onto said first member (49).

2. Injection cartridge according to Claim 1, characterised in that the first member (49) is made from a glass of medical quality of type I.

3. Injection cartridge according to Claim 1, characterised in that the second member (50) is made from a plastics material having the following features:
- of withstanding a sterilisation operation at high temperature,
- of being approved for pharmaceutical application,
- of being insensitive to temperature variations,
- of being translucent, even transparent and possibly coloured.

4. Injection cartridge according to Claim 1, characterised in that the first member (49), as a whole of substantially cylindrical shape, comprises at each end an orifice (29, 30), one (30) of the orifices having a diameter substantially equivalent to the diameter of a rod (17) of the percussion member, whereas the other (29), of small diameter, in particular of the order of several tenths of a millimetre, serves as a nozzle.

5. Injection cartridge according to Claim 1, characterised in that the means (51) allowing the engagement and interconnection are formed at the time of the covering operation of moulding-on through the intermediary of a plurality of lugs (52) projecting radially and arranged around the diameter of said cartridge (28), these lugs cooperating at the level of the bayonets provided at the end (53) of the injection device.

6. Injection cartridge according to one of the preceding Claims, characterised in that the end (54) of the cartridge (28) is covered by a cap (55) provided at its centre with a gasket (56) made of elastomeric material, in particular made of silicone.

7. Injection cartridge according to Claim 6, characterised in that the cap (55) has side walls (57) which grip the end of said cartridge (28), said walls (57) being provided at their ends with areas (58) in relief allowing their clipping in recesses (59) provided on the outer side walls of said cartridge (28), consequently ensuring the retention of the cap (55) on the cartridge.

8. Injection cartridge according to one of Claims 6 or 7, characterised in that the upper wall (60) of the cap (55) forms a gripping area which enables the user to position the lugs (52) of the cartridge (28) in line with the bayonets of the injection device.

9. Injection cartridge according to Claim 6, characterised in that the elastomeric gasket (56) is obtained at the time of a simultaneous operation for moulding the cap (55).

10. Injection cartridge according to Claim 6, characterised in that the elastomeric gasket (56) is connected and force-fitted in an orifice (61) provided on the inner wall (62) of the cap (55).

11. Injection cartridge according to one of the preceding Claims, characterised in that it comprises a plurality of gaps in the plastics material, provided in the thickness of the plastic moulding-on of the cartridge (28), in order to produce apertures (63) in order to improve the visibility at the level of the first member (49).
